# EUROPEAN PATENT SPECIFICATION

(11) **EP 4 258 904 B1**
(45) Date of publication and mention of the grant of the patent: **20.11.2024**
(21) Application number: 21831025.8
(22) Date of filing: 09.12.2021
(51) Int. Cl.: A23L 33/21, A23L 33/00, A61P 1/10

(54) **FIBRE MIXTURE FOR YOUNG CHILDREN**
BALLASTSTOFFMISCHUNG FÜR KLEINKINDER
MÉLANGE DE FIBRES POUR ENFANTS EN BAS ÂGE

(30) Priority: 09.12.2020 EP 20212719
(43) Date of publication of application: 18.10.2023
(73) Proprietor: N.V. Nutricia, 2712 HM Zoetermeer (NL)
(72) Inventor: BOURITIUS, Houkje, 3584 CT Utrecht (NL); TIMS, Sebastian, 3584 CT Utrecht (NL)
(74) Representative: Nederlandsch Octrooibureau
(86) International application number: PCT/EP2021/085057
(87) International publication number: WO 2022/122958

(56) References cited:
- EP-A1- 0 756 828
- WO-A1-2006/096882
- WO-A1-2009/075573
- KOKKE FREDDY T M ET AL: "Downloaded from http A Dietary Fiber Mixture versus Lactulose in the Treatment of Childhood Constipation: A Double-blind Randomized Controlled Trial", 1 January 2008 (2008-01-01), XP055798586, Retrieved from the Internet <URL:https://journals.lww.com/jpgn/Fulltext/2008/11000/A_Dietary_Fiber_Mixture_versus_Lactulose_in_the.11.aspx> [retrieved on 20210423]
- LE BAO ET AL: "Prebiotic Potential and Anti-Inflammatory Activity of Soluble Polysaccharides Obtained from Soybean Residue", FOODS, vol. 9, no. 12, 6 December 2020 (2020-12-06), pages 1808, XP055798260, DOI: 10.3390/foods9121808
- TAKAHASHI TARO ET AL: "Physiological Effects of Water-soluble Soybean Fiber in Rats", vol. 63, no. 8, 1 January 1999 (1999-01-01), JP, pages 1340 - 1345, XP055798246, ISSN: 0916-8451, Retrieved from the Internet <URL:https://www.tandfonline.com/doi/pdf/10.1271/bbb.63.1340> DOI: 10.1271/bbb.63.1340
- LIN DERONG ET AL: "Study on the functional properties and structural characteristics of soybean soluble polysaccharides by mixed bacteria fermentation and microwave treatment", INTERNATIONAL JOURNAL OF BIOLOGICAL MACROMOLECULES, ELSEVIER BV, NL, vol. 157, 25 April 2020 (2020-04-25), pages 561 - 568, XP086191440, ISSN: 0141-8130, [retrieved on 20200425], DOI: 10.1016/J.IJBIOMAC.2020.04.133
- MA YAN ET AL: "Effects of soybean oligosaccharides on intestinal microbial communities and immune modulation in mice", vol. 24, no. 1, January 2017 (2017-01-01), pages 114 - 121, XP029866445, ISSN: 1319-562X, Retrieved from the Internet <URL:https://doi.org/10.1016/j.sjbs.2016.09.004> DOI: 10.1016/J.SJBS.2016.09.004
- AXELROD CARA ET AL: "The Role of Fiber in the Treatment of Functional Gastrointestinal Disorders in Children", NUTRIENTS, vol. 10, no. 11, 3 November 2018 (2018-11-03), pages 1650, XP055798247, DOI: 10.3390/nu10111650
- ANONYMOUS: "FIBRIM Soy Fiber", 28 October 2020 (2020-10-28), XP055798641, Retrieved from the Internet <URL:https://web.archive.org/web/20201028090536/https://www.dupontnutritionandbiosciences.com/products/fibrim-soy-fiber.html> [retrieved on 20210423]
- ARCIA P.L. ET AL: "Inulin blend as prebiotic and fat replacer in dairy desserts: Optimization by response surface methodology", JOURNAL OF DAIRY SCIENCE, vol. 94, no. 5, 1 May 2011 (2011-05-01), US, pages 2192 - 2200, XP055798644, ISSN: 0022-0302, DOI: 10.3168/jds.2010-3873

## Description

### FIELD OF THE INVENTION

The present invention is in the field of mixtures of dietary fibres that have a beneficial effect in the prevention of constipation, in particular for young children.

### BACKGROUND OF THE INVENTION

Fibres are an important part of the diet. They have many beneficial effects, in particular on gut health, and in particular on prevention of constipation.

For infants a source of dietary fibres are the human milk oligosaccharides as found in human milk. Infant formulas have been designed to mimic functionally and/or structurally the human milk oligosaccharides. For example, mixtures of galacto-oligosaccharides (GOS) and polyfructose or long-chain fructo-oligosaccharides (IcFOS) were found to reduce the number of hard stools in infants (Moro et al., J Pediatr Gastroenterol Nutr. 2002;34(3):291-295).

In adult nutrition the dietary fibres are mainly derived from plant sources and consist of a mixture of soluble and insoluble fibre, a mixture of indigestible poly- and oligosaccharides, and fermentable and non-fermentable fibres. EP 0 756 828 describes a fibre mix with a composition representing the dietary fibre in a typical adult Western diet.

WO 2009/075573 discloses a mixture suitable for young children as a transition between fibres consumed during infancy and during adulthood. In a clinical study with paediatric patients a good effect on treating constipation was observed.

WO 2011/060123 relates to nutritional compositions comprising a fructo-oligosaccharide (FOS) in an amount of 35 to 44% by weight; a polysaccharide that is not a partially hydrolyzed guar gum, such as for example an arabinogalactan, in an amount of 50% to 38% by weight; and inulin in an amount of 12% to 24% by weight.

However, still further improvements can be made in the design in optimal fibres mixes to treat or prevent or reduce constipation, in particular in young children.

### SUMMARY OF THE INVENTION:

The inventors performed faecal fermentation experiments comparing a prior art fibre mixture comprising inulin, beta-galacto-oligosaccharides, resistant starch and insoluble soy polysaccharides with a new fibre mix wherein the insoluble soy polysaccharides were replaced by soluble soy polysaccharides. Surprisingly, the new fibre mix with 4 fibres including the soluble soy polysaccharides resulted in a higher amount of short chain acids produced, and in particular a significantly higher amount of butyric acid produced and a lower amount of propionic acid when compared with the fibre mix of 4 fibres containing insoluble soy polysaccharides. Since an increased amount of butyric acid in the colon in toddlers and young children is known to be beneficial, and since there is an inverse relationship between the colonic butyrate levels and constipation, and propionate having an opposite effect, this is indicative for an improved effect of the new fibre mix on constipation, and its suitability in particular for young children.

### DETAILED DESCRIPTION OF THE INVENTION:

The present invention thus concerns a fibre mixture consisting of a) beta-galactooligosaccharides, b) inulin, c) resistant starch and d) soluble soy polysaccharides, wherein the weight ratio beta-galactooligosaccharides : inulin : resistant starch : soluble soy polysaccharides is about 38 : 38 : 4 : 20. The present invention also concerns a nutritional composition when in liquid form comprising 0.4 - 10 g per 100 ml of the fibre mixture according to the invention, or when in powder form, comprising 3 - 45 g per 100 g dry weight of the fibre mixture according to the invention, wherein the fibres present in the nutritional composition consist of the fibre mixture according to the invention.

The present invention also concerns a fibre mixture according to the invention, or a nutritional composition comprising a fibre mixture according to the invention, for use in treating or preventing or reducing the risk of constipation in a human subject.

The present invention also concerns a fibre mixture according to the invention, or a nutritional composition comprising a fibre mixture according to the invention, for non-therapeutic use in softening stool and/or decreasing stool consistency and/or maintaining a soft stool in a human subject compared to stool consistency resulting from the fibre mixture wherein the soluble soy polysaccharides are replaced by insoluble soy polysaccharides, wherein stool consistency is quantified by using the Bristol Stool Form Scale (Lewis & Heaton, Scand J Gastroenterol 1997, 32:920-924).

The present invention also concerns a fibre mixture according to the invention, or a nutritional composition comprising a fibre mixture according to the invention, for non-therapeutic use in increasing intestinal butyrate levels and/or for decreasing intestinal propionate levels, preferably for increasing intestinal butyrate levels, compared to the levels of butyrate and/or propionate resulting from the fibre mixture wherein the soluble soy polysaccharides are replaced by insoluble soy polysaccharides.

The invention is defined by the appended claims.

### Fibre mixture

Fibres are non-digestible carbohydrates. Non-digestible carbohydrates are carbohydrates that are resistant to digestion and absorption in the human stomach and small intestine and enter the colon intact. So, compounds like lactose, maltose, glucose, standard maltodextrin and standard starch are regarded as digestible. Fibres can be soluble or insoluble in water. The term "soluble" as used herein, when having reference to a non-digestible carbohydrate, means that the substance is water soluble according to the method described by L. Prosky et al., J. Assoc. Off. Anal. Chem. 71, 1017-1023 (1988).

If a fibre, i.e. non-digestible carbohydrate, is not water soluble according to the method described by Prosky, the fibre is considered insoluble. Fibres can be fermentable in the colon, or non-fermentable. The term "fermentable" refers to the capability to undergo (anaerobic) breakdown by micro-organisms in the lower part of the gastro-intestinal tract, e.g. colon, to smaller molecules, in particular short chain fatty acids and lactate. The fermentability may be determined by the method described in Am. J. Clin. Nutr. 53, 1418-1424 (1991). Fibres can be as short as a dimer of 2 monomeric carbohydrate moieties, but can also have an average degree of polymerization well above 1500. Fibres with a degree of polymerization 2 - 10 are considered oligomers, whereas fibres with a degree of polymerization above 10 are considered polymers.

The present fibre mixture consists of beta-galactooligosaccharides, inulin, resistant starch and soluble soy polysaccharide in a weight ratio of about 38 : 38 : 4 : 20.

This ratio of these four fibres ensures an optimal balance and/or interaction between the different types of fibres and their specific beneficial effect, such as colonic butyrate formation, microbiota composition and anti-constipation effect.

### Soluble soy polysaccharides

The present fibre mixture comprises soluble soy polysaccharide. This can also be referred to as soluble soybean polysaccharides.

Soluble soy polysaccharide is typically extracted from soybean cotyledons and has a pectin like structure. However it is different in composition from other commercially available pectins as from fruit. Soluble soy polysaccharide is also different in structure form the insoluble soy polysaccharides. Typically the structure is as follows:
i) The starting non-reducing-end backbone composed of a galacturonan chain: [-4)-α-D-GalA-1-]m (m = 7-9).
ii) a first intermediate backbone composed of a rhamnogalacturonan chain: [-4)-α-D-GalA-(1→2)-α-L-Rha -(1→4)-α-D-GalA-(1-]n (n = 15-100);
   the first intermediate backbone carries the following side chains:
   - homoarabinan side chains: [-3 or 5)-α-L-Ara-1-]o (o = undefined)
   - homogalactan side chains: [-4)-b-D-Gal-1-]p (p = undefined)
   - galacturonans with side chains composed of xylans
   - galactans chain branched by neutral sugars (Ara, Xyl, Fuc, Glc)
iii) a second intermediate backbone composed of a rhamnogalacturonan chain: [-2)-α-L-Rha-(1→4)-α-D-GalA-(1]k→2-a-L-Rha-(1→4)-α-D-GalA-(1-] (k =4 -10)
iv) a terminal reducing-end backbone composed of a galacturonan chain: [-4)-α-D-GalA-1-].

Preferably the soluble soy polysaccharide has a degree of polymerization in the range from 50 - 500 and is a branched polymer. The detailed structure of the main component of soybean hemicelluloses has a molecular weight of 550×10³g/mol and consists of two types of units in the main backbone: a short-chain homogalacturonan (degree of polymerisation, DP, 4-10) and a long-chain rhamnogalacturonan (repeating units 15, 28, 100). The side chains are composed of galactans (DP, 43-47) branched with fucose and arabinose residues, and arabinans; these arabinans are linked to rhamnose residues in the rhamnogalacturonan. Some galacturonic acids are modified xylosyl oligosaccharides (DP, 4-7). Several varieties of commercially available soybean hemicellulose exist but they all are very similar in composition and are composed of the same types of sugars. They have about 19 to 20 % galacturonic acid, about 46 to 50 % galactose, about 18 to 23 % arabinose, about 5 to 5.5 % rhamnose, about 1 to 3 % fucose, about 3 to 3 % xylose and about 1 to 2 % glucose.

Soluble soy polysaccharides are commercially available, for example-Soyafibe-S-Ca100 (Fuji Oil Co, Ltd) Soyafibe S-DN and Soya-Up M 800 also known as additive E 426 (as defined in EU 231/2012). Nakamura et al, 2001 Biosci Biotech Biochem 65 2249-2258 give further information on soluble soy polysaccharides.

A sufficient amount of soluble soy polysaccharide is advantageous for children, in particular paediatric patients and/or constipated children, since the inventors have shown that when present in a mix with galacto-oligosaccharides, inulin and resistant starch, it is shown to increase short chain fatty acid formation, and in particular butyrate production, when compared to a mix of fibres that comprises insoluble soy fibre. This is beneficial for young children and beneficial for preventing or treating constipation. The use of soluble soy polysaccharide together with the other fibres according to the invention results in an intestinal microbiota which is intermediate, regarding bifidobacteria, between infants and adult subjects. Without the presence of soluble or insoluble soy polysaccharides the microbiota would have too high amounts of proteobacteria and too low amounts of bifidobacteria. A too high amount of soluble soy polysaccharides will result in an imbalance with the other fibres according the invention.

Lin et al. Int J Biol Macromol 2020, 157:561-568 describe that growth of lactobacilli is stimulated by soluble soy polysaccharides. Lactobacilli are known to produce lactic acid and sometimes acetic acid. Chen et al. Animal Feed Science and Technology 2014, 195:101-111 describe that soy fiber fermentation results in a significantly increased amount of acetic acid, when compared to a control, but the effect on butyric acid and propionic acid is not significantly different. It is thus unexpected that in the mix of 4 fibres of the present invention, an increase of butyrate is observed when replacing insoluble soy polysaccharides with soluble soy polysaccharides.

### Beta-galactooligosaccharides

Betagalacto-oligosaccharides as used in the present invention refers to oligosaccharides composed of more than 50%, preferably more than 65% galactose units based on total monomeric units of the etagalacto-oligosaccharides, with an average degree of polymerization (DP) of 2 - 10, in which at least 50%, more preferably at least 75%, even more preferably at least 90%, of the galactose units are linked together via a beta-glycosidic linkage, preferably a beta-1,4-glycosidic linkage, a beta-1,6-glycosidic linkage and/or a beta-1,3-glycosidic linkage. The average DP is preferably in the range of 3 - 6. A glucose unit may be present at the reducing end of the chain of galactose units. Beta-galactooligosaccharides are sometimes also referred to as trans-galactooligosacchariodes (TOS). Beta-galactooligosaccharides can be analyzed according to AOAC method 2001.02. A suitable source of beta-galactoologosaccharides is Vivinal^{®}GOS (commercially available from Borculo Domo Ingredients, Zwolle, Netherlands). Other suitable sources are Oligomate^{®} (Yakult), Cupoligo^{®} (Nissin) and Bi2muno^{®} (Classado).

Beta-galactooligosaccharides are reminiscent to human milk oligosaccharides in that human milk oligosaccharides also comprise beta-glycosidic linkages and comprise galactose as a monomeric unit. A high amount of beta-galactooligosaccharides is advantageous for children, in particular toddlers, in particular paediatric patients and/or constipated children, since it favourably stimulates the intestinal bifidobacteria, the intestinal production of the organic acids lactate and acetate and stimulates the immune system. The use of beta-galactooligosaccharides together with the other fibres according to the invention results in an intestinal microbiota rich in bifidobacteria, which is beneficial for young children. The use of beta-galactooligosaccharides together with the other fibres according to the invention results in an intestinal microbiota rich in bifidobacteria, which is beneficial for treating or preventing constipation. A too high amount of beta-galactooligosaccharides will result in an imbalance between beta-galactooligosaccharides with the other fibres of the invention. A too high amount of beta-galactooligosaccharides will result in a too fast and high fermentation in the beginning of the colon

### Inulin

Inulin as used in the present invention refers to carbohydrates composed of more than 50%, preferably more than over 65% fructose units based on total monomeric units of the inulin, in which at least 50 %, more preferably at least 75%, even more preferably at least 90%, of the fructose units are linked together via a beta-glycosidic linkage, preferably a beta-2,1-glycosidic linkage. A glucose unit may be present at the reducing end of the chain of fructose units. Preferably the composition comprises long chain inulin with an average DP above 20. A suitable source of inulin is for example Orafti^{®}HP (commercially available from Beneo, Belgium).

A sufficient amount of inulin is advantageous for children, in particular paediatric patients and/or constipated children since it favourably stimulates the intestinal bifidobacteria and/or the intestinal production of the organic acids. The use of inulin together with beta-galactooligosaccharides has a synergistic effect in respect of stimulation of bifidobacteria and production of organic acids. The use of inulin together with the other fibres according the invention results in an intestinal microbiota which is rich in bifidobacteria, and low in proteobacteria, which is beneficial for young children and is beneficial in treating or preventing constipation. A too high amount of inulin will result in an imbalance between inulin with the other fibres according the invention. A too high amount of inulin will result in excessive gas formation, bloating and flatulence. Inulin can be analyzed according to AOAC method 997.08. The advantageous effects of inulin in combination with the other fibres according to the invention is best achieved when the inulin is long-chain inulin. In a preferred embodiment according to the present invention, the inulin has an average degree of polymerization above 15, preferably above 20.

### Resistant starch

The fibre mixture according to the present invention comprises resistant starch. Resistant starch is a non-digestible alpha-glucan. Resistant starch relates to non-digestible carbohydrate polymers made up of at least 80% glucose monomers, based on total monomers of the resistant starch, preferably at least 85%, which are bound together for more than 50% via alpha-glycosidic linkages, and which are resistant to digestion and absorption in the human stomach and small intestine and enter the colon intact.

Resistant starch can be determined according to the method described by McCleary and Monaghan (2002) J AOAC Int 85, 665-675. Resistant starch includes starch that is physically inaccessible to the digestive enzymes in the stomach and small intestine, starch that is inaccessible to enzymes due due to its conformation, e.g. its natural granular form, such as uncooked potato starch, green banana flour and high amylase corn, starch that is formed when starch-containing foods are cooked and cooled (retrograded starch), and starch that is chemically modified to resist digestion. Preferably the resistant starch is retrograded starch and/or starch that is chemically modified to resist digestion, most preferably retrograded starch, since this gives a more stable product. A suitable source of resistant starch is Novelose^{®} 330 (Ingredion).

Preferably the fibre mixture comprises resistant starch in the form of high amylose starch, preferably from corn, cassava or potato, more preferably from corn. Preferably the resistant starch is a retrograded starch (RS3). More preferably the resistant starch is a retrograded resistant high amylose starch, preferably from corn.

Preferably the fibre mixture comprises resistant starch in the form of non-digestible dextrin. The terms "non-digestible dextrin", "indigestible polydextrin", "indigestible dextrin", "indigestible maltodextrin", "polydextrose", "resistant polydextrin", "resistant maltodextrin", "pyrodextrin" or "resistant dextrin" are used interchangeably in the present invention and refer to non-digestible carbohydrates which have a DP in the range of 3 - 50, preferably in the range of 4 - 20 and in which the monomeric units are at least 80 %, preferably at least 85 % originating from glucose, based on the total of monomeric units of the resistant starch, and comprising at least 50%, preferably at least 80% alpha-glycosidic linkages. The average degree of polymerization is preferably in the range from 10 - 16 monosaccharide units per molecule. In a preferred embodiment, the indigestible polydextrins are randomly branched and comprise alpha-(1 ,4)- and alpha-(1 ,6)-glycosidic linkages. Additionally up to about 11% other monomers such as sorbitol and citric acid may be present. Indigestible dextrin can be obtained by heating and alpha-amylase treatment of starch. Alternatively, polydextrose can be obtained by heating (under vacuum) dextrose with a food acid catalyst and sorbitol and purifying the resulting water-soluble polymer. Indigestible dextrin is for example available under the tradename "Fibersol 2^{®}" from Matsutami Inductries or Litesse^{®} from Danisco. A suitable method to determine the non-digestible glucan prepared by vacuum thermal polymerization of glucose, using food acid and sorbitol (e.g. polydextrose, Litesse^{®}) is AOAC method 2000.11. A suitable way to determine the non-digestible glucan derived by heat and enzyme treatment of starch (e.g. pyrodextrin, Fibersol^{®}) is AOAC method 985.29.

A sufficient amount of resistant starch is advantageous for children, in particular toddlers, in particular paediatric patients and/or constipated children since it advantageously results in a fermentation at the more distal part of the colon. The use of resistant starch advantageously results in the formation of butyrate, an organic acid which is a substrate of the intestinal enterocytes. The use of resistant starch together with the other fibres according the invention results in an intestinal microbiota which is intermediate, regarding bifidobacteria, between infants and adult subjects. A too high amount of resistant starch will result in an imbalance with the other fibres according the invention. A too high amount of resistant starch may result in unfavourable product characteristics such as a high viscosity and precipitations.

### Nutritional composition

In one embodiment the present invention also concerns a nutritional composition comprising the fibre mixture according to the present invention. In one embodiment the present nutritional composition is a liquid. In one embodiment, preferably the present nutritional composition is a ready-to-feed composition. Preferably the composition is administered orally. In one embodiment the present nutritional composition is in a powdered form, which can be reconstituted with water to form a liquid. In the context of the present invention the terms 'powder' and 'dry' are used interchangeably.

The nutritional composition according to the invention comprises 0.4 - 10 g of the present fibre mixture per 100 ml, preferably 0.5 - 10 g of the present fibre mixture per 100 ml, preferably 0.6 - 8 g, more preferably 1 - 5 g and even more preferably 1.2 - 3 g of the present fibre mixture per 100 ml nutritional composition. When in powder form, the nutritional composition according to the invention comprises 3 - 45 g of the present fibre mixture per 100 g dry weight, preferably 4 - 40 g, more preferably 7 - 25 g and even more preferably 8-20 g of the present fibre mixture per 100 g dry weight of the nutritional composition. Based on calories, preferably the nutritional composition according to the invention comprises 0.6 - 15 g of the present fibre mixture per 100 kcal, preferably 0.9 - 12 g, more preferably 1.5 - 7.5 g and even more preferably 1.8 - 5 g of the present fibre mixture per 100 kcal nutritional composition.

The quantity of fibres promotes the advantageous effects of these fibres in the gastro-intestinal tract yet is suitable for children and minimizes the risk of unwanted side effects such as bloating, abdominal pain, flatulence and/or a feeling of satiety. The amount of fibre can suitably be determined according to McCleary, Anal Bioanal Chem 2007, 389:291-308. This method suitably determines total fibre including resistant starch and non-digestible oligosaccharides. For the purpose of the present invention the caloric density of the fibres is set at 2 kcal per gram.

The nutritional composition according to the invention preferably comprises digestible carbohydrate, lipid and protein. The lipid preferably provides 20 - 55% of the total calories, the protein preferably provides 5 - 15% of the total calories and the digestible carbohydrate preferably provides 30 - 75% of the total calories of the nutritional composition. Preferably nutritional composition according to the invention comprises lipid providing 25 - 50 % of the total calories, protein providing 6 - 13% of the total calories and digestible carbohydrate providing 40 - 65% of the total calories of the nutritional composition. More preferably the present nutritional composition comprises lipid providing 30 - 45 % of the total calories, protein providing 7 - 10% of the total calories and digestible carbohydrate providing 45 - 55% of the total calories of the nutritional composition.

In order to meet the caloric requirements of the child, the nutritional composition according to the invention preferably comprises 45 - 100 kcal per 100 ml, more preferably 50 - 75 kcal per 100 ml, even more preferably 60 -70 kcal 100 ml. This caloric density ensures an optimal ratio between water and calorie consumption and this balance is important for preventing constipation. The amount of calories is the sum of the calories provided by the protein, the lipid, and the digestible carbohydrate and fibres.

The nutritional composition preferably comprises 1.5 - 4.5 g lipid per 100 ml, more preferably 2.0 - 4.0 g per 100 ml, more preferably 2.5 - 3.5 g per 100 ml. Based on dry weight, the nutritional composition preferably comprises 10 - 35 g lipid per 100 g, more preferably 14 - 30 g per 100 g, more preferably 16 - 25 g lipid per 100 g dry weight of the nutritional composition. Based on calories, the nutritional composition preferably comprises 2.0 - 7.0 g lipid per 100 kcal, more preferably 2.5 - 6.0 g per 100 kcal, more preferably 3.0 - 5.0 lipid g per 100 kcal of the nutritional composition. The lipid preferably provides 20 - 55%, more preferably 25 - 50%, more preferably 30 - 45% of the total calories of the present nutritional composition.

The amount of saturated fatty acids is preferably below 45 wt.% based on total lipids more preferably below 25 wt.%. The concentration of monounsaturated fatty acids preferably ranges from 30 to 65% based on weight of total fatty acids. The concentration of polyunsaturated fatty acids preferably ranges from 15 to 60% based on weight of total fatty acids. Preferably the nutritional composition comprises the n-6 polyunsaturated fatty acid linoleic acid (LA) and the n-3 polyunsaturated fatty acid alpha-linolenic acid (ALA). LA and ALA are essential fatty acids and important for healthy growth and development of children. Preferably the nutritional composition comprises long chain poly-unsaturated fatty acids (LC-PUFA). LC-PUFA are defined in the present invention as fatty acids or acyl chains with two or more double bonds and a chain length of 20 or above. Preferably the nutritional composition comprises docosahexaenoic acid (DHA) and/or eicosapentaenoic acid (EPA). DHA and EPA are n-3 LC-PUFA which play a role in preventing intestinal inflammation and intestinal pain, thereby further improving the intestinal health in subjects suffering from or at risk of constipation.

The nutritional composition preferably comprises 0.8 - 2.5 g protein per 100 ml, more preferably 1.0 - 2.0 g per 100 ml, more preferably 1.2 - 1.7 g per 100 ml nutritional composition. Based on dry weight, the nutritional composition preferably comprises 6 - 20 g protein per 100 g, more preferably 7 - 16 g per 100 g, more preferably 8 - 12 g protein per 100 g dry weight of the nutritional composition. Based on calories, the nutritional composition preferably comprises 1.2 - 4.0 g protein per 100 kcal, more preferably 1.5 - 3.3 g per 100 kcal, more preferably 1.7 - 2.5 g protein per 100 kcal of the nutritional composition. The protein preferably provides 5 - 15%, more preferably 6 - 13% even more preferably 7 - 10% based on total calories of the composition.

Protein is to be taken as the sum of proteins, peptides and free amino acids. The amount of protein can be calculated according to the amount of nitrogen multiplied by 6.25.

The present nutritional composition preferably comprises casein and/or whey proteins. Preferably the weight ratio casein:whey protein is 0:100 to 90:10, more preferably 20:80 to 90:10, more preferably 40:60 to 80:20.

The nutritional composition preferably comprises 5 - 15 g digestible carbohydrates per 100 ml, more preferably 6 - 15 g per 100 ml, more preferably 7 - 10 g per 100 ml nutritional composition. Based on dry weight, the nutritional composition preferably comprises 35 - 84 g digestible carbohydrates per 100 g, more preferably 45 - 80 g per 100 g, more preferably 55 - 70 g digestible carbohydrates per 100 g dry weight of the nutritional composition. Based on calories, the nutritional composition preferably comprises 7 - 20 g digestible carbohydrates per 100 kcal, more preferably 10 - 18 g per 100 kcal, more preferably 12 - 15 g digestible carbohydrates per 100 kcal of the nutritional composition. The digestible carbohydrates preferably provides 30 - 75%, more preferably 40 - 65%, more preferably 45 - 55 % of the total calories of the present nutritional composition.

Preferably the composition comprises at least one digestible carbohydrate selected from the group consisting of lactose, maltodextrin, digestible starch, saccharose, glucose, and maltose, more preferably lactose

The fibres present in the nutritional composition consist of the fibre mixture according to the present invention.

In terms of individual fibres the nutritional composition according to the invention preferably comprises at least 0.08 g soluble soy polysaccharides per 100 ml, more preferably at least 0.1 g, even more preferably at least 0.2 g soluble soy polysaccharides per 100 ml. Based on dry weight, the nutritional composition according to the invention preferably comprises at least 0.5 g soluble soy polysaccharides per 100 g, more preferably at least 0.7 g, even more preferably at least 1.5 g soluble soy polysaccharides per 100 g. Based on calories, the nutritional composition according to the invention preferably comprises at least 0.12 g soluble soy polysaccharides per 100 kcal, more preferably at least 0.15 g, even more preferably at least 0.3 g soluble soy polysaccharides per 100 kcal.

The nutritional composition according to the invention preferably comprises less than 2 g soluble soy polysaccharides per 100 ml, more preferably less than 1.5 g, even more preferably less than 1.0 g soluble soy polysaccharides per 100 ml. Based on dry weight, the nutritional composition according to the invention preferably comprises less than 10 g soluble soy polysaccharides per 100 g, more preferably less than 7.5 g, even more preferably less than 5.0 g soluble soy polysaccharides per 100 g. Based on calories, the nutritional composition according to the invention preferably comprises less than 3 g soluble soy polysaccharides per 100 kcal, more preferably less than 2.5 g, even more preferably less than 1.5 g soluble soy polysaccharides per 100 kcal.

Furthermore, the nutritional composition according to the invention preferably comprises at least 0.15 g beta-galactooligosaccharides per 100 ml, more preferably at least 0.2 g, even more preferably at least 0.4 g beta-galactooligosaccharides per 100 ml. Based on dry weight, the nutritional composition according to the invention preferably comprises at least 1.0 g beta-galactooligosaccharides per 100 g, more preferably at least 1.5 g, even more preferably at least 3.0 g beta-galactooligosaccharides per 100 g. Based on calories, the nutritional composition according to the invention preferably comprises at least 0.2 g beta-galactooligosaccharides per 100 kcal, more preferably at least 0.3 g, even more preferably at least 0.6 g beta-galactooligosaccharides per 100 kcal.

The nutritional composition according to the invention preferably comprises less than 4 g beta-galactooligosaccharides per 100 ml, more preferably less than 3 g, even more preferably less than 2 g beta-galactooligosaccharides per 100 ml. Based on dry weight, the nutritional composition according to the invention preferably comprises less than 20 g beta-galactooligosaccharides per 100 g, more preferably less than 15 g, even more preferably less than 10 g beta-galactooligosaccharides per 100 g. Based on calories, the nutritional composition according to the invention preferably comprises less than 6 g beta-galactooligosaccharides per 100 kcal, more preferably less than 4.5 g, even more preferably less than 3 g beta-galactooligosaccharides per 100 kcal.

Furthermore, the nutritional composition according to the invention preferably comprises at least 0.15 g inulin per 100 ml, more preferably at least 0.2 g, even more preferably at least 0.4 g inulin per 100 ml. Based on dry weight, the nutritional composition according to the invention preferably comprises at least 1.0 g inulin per 100 g, more preferably at least 1.5 g, even more preferably at least 3.0 g inulin per 100 g. Based on calories, the nutritional composition according to the invention preferably comprises at least 0.2 g inulin per 100 kcal, more preferably at least 0.3 g, even more preferably at least 0.6 g inulin per 100 kcal.

The nutritional composition according to the invention preferably comprises less than 4 g inulin per 100 ml, more preferably less than 3 g, even more preferably less than 2 g inulin per 100 ml. Based on dry weight, the nutritional composition according to the invention preferably comprises less than 20 g inulin per 100 g, more preferably less than 15 g, even more preferably less than 10 g inulin per 100 g. Based on calories, the nutritional composition according to the invention preferably comprises less than 6 g inulin per 100 kcal, more preferably less than 4.5 g, even more preferably less than 3 g inulin per 100 kcal.

Furthermore, the nutritional composition according to the invention preferably comprises at least 0.015 g resistant starch per 100 ml, more preferably at least 0.02 g, even more preferably at least 0.040 g resistant starch per 100 ml. Based on dry weight, the nutritional composition according to the invention preferably comprises at least 0.1 g resistant starch per 100 g, more preferably at least 0.15 g, even more preferably at least 0.3 g resistant starch per 100 g. Based on calories, the nutritional composition according to the invention preferably comprises at least 0.02 g resistant starch per 100 kcal, more preferably at least 0.03 g, even more preferably at least 0.04 g resistant starch per 100 kcal.

The nutritional composition according to the invention preferably comprises less than 0.4 g resistant starch per 100 ml, more preferably less than 0.3 g, even more preferably less than 0.2 g resistant starch per 100 ml. Based on dry weight, the nutritional composition according to the invention preferably comprises less than 2.0 g resistant starch per 100 g, more preferably less than 1.5 g, even more preferably less than 1.0 g resistant starch per 100 g. Based on calories, the nutritional composition according to the invention preferably comprises less than 0.6 g resistant starch per 100 kcal, more preferably less than 0.45 g, even more preferably less than 0.3 g resistant starch per 100 kcal.

In one embodiment it is preferred that the nutritional composition does not comprise probiotic bacteria. Not administering probiotic bacteria is considered to help in a sustained relieve of constipation.

Preferably the nutritional composition comprises vitamins, minerals and trace elements and other micronutrients in recommended daily amounts as known in the art and according to international guidelines.

The osmolarity of the present nutritional composition is preferably between 150 and 700 mOsmol/l, more preferably 200 to 400 mOsmol/l. This osmolarity advantageously reduced gastro-intestinal stress, results in an optimal balance between water and nutrient uptake, which is beneficial for children suffering from or at risk of constipation.

### Application

The occurrence of constipation in toddlers and children can be high and was reported to be 10% in the second year of life (Loehning-Baucke, J Pediatr 2005; 146:359-363). Functional constipation, also known as chronic idiopathic constipation, has a large impact on the quality of life and healthcare costs. Lactulose or poly-ethylene glycol are commonly prescribed laxatives in case of chronic childhood constipation. However, these are fibres not naturally occurring in food, having their effect mainly in the proximal colon and having no additional benefits for the child.

The new mixture of 4 fibres including soluble soy polysaccharides, was found to have an optimal effect when looking at the effects on microbiota, the amount of SCFA formed, and the amount of butyrate formed. Without wishing to be bound by theory, an increased amount of colonic SCFA, in particular including butyrate, will stimulate the colonic smooth muscle contractility, which advantageously results in treating or preventing constipation. Butyric acid may increase the peristaltic efficiency and may improve the regulation of intestinal neurotransmission. Constipation is often associated with a decreased intestinal butyrate level, see for example Ge et al. Sci Rep 2017, 7:441, DOI:10.1038/s41598-017-00612-y; Pituch et al. Prz Gastroenterol 2013, 8(5):295-298; Zhuang yet al. Molecular Nutrition Food Research 2019 https://doi.org/10.1002/mnfr.201801187; He et al. J Cell Mol Med 2020, 24:9349-9361; Yusuf et al. Syst Rev in Pharmacy 2020, 11 :587-592; Chassard et al. Aliment Pharmacol Therap 2012, 35:818-838. Propionate on the other hand, appears to have an opposite effect. Hurst et al. Neurogastroenterol Motil 2014, 26:1586-1596 show that butyrate and propionate have a differential effect on the motility of the colon, with butyrate increasing the frequency of full length propagations, and propionate blocking full and short propagations. Interestingly, Farup et al. BMC Gastroenterolgy 2016, 16:51 show that in IBS subjects the difference of faecal propionic acid and butyric acid had the best diagnostic properties, including for subjects suffering from the constipation form of IBS. In IBS patients, compared to healthy subjects, the amount of butyrate was decreased and amount of propionic acid was increased, with the "propionic acid minus butyric acid: values being significantly higher in the IBS-C group.

The effects on microbiota observed with the new mixture of 4 fibres with soluble soy polysaccharide, shows that the microbiota is comparable with both mixes, not showing statistically significant differences on for example level of Bifiobacteria. In short, the mixture of fibres of the present invention beneficially affects the bowel health of human subjects, in particular children of 1 to 12 years. The new mixture of fibres results in an improved formation of short chain fatty acids, in particular butyric acid, without negatively effecting the intestinal microbiota, when compared with the prior art fiber mixture. The new fiber mixture also showed an improved effect on the formation of short chain fatty acids, in particular butyrate, when compared with lactulose, a well-known laxative. The fibre mixture of the present invention, preferably included in a nutritional composition, is therefore preferably used to treat and/or prevent constipation, more preferably to treat constipation in a constipated child. The fibre mixture of the present invention, preferably included in a nutritional composition, is therefore preferably used to soften stools and/or to decrease stool consistency, more preferably in a constipated child. The fibre mixture of the present invention, preferably included in a nutritional composition, is therefore preferably used to maintain a soft stool and/or to maintain a desired stool consistency, more preferably in a child that is at risk of constipation. Effects on softening of stool or decreasing stool consistency can be suitably quantified by using the Bristol Stool Form Scale (Lewis & Heaton, Scand J Gastroenterol 1997, 32:920-924). Types 3-5 are considered a soft stool, or a stool with an appropriate consistency. Types 1-2 are hard stools, or stools with too high consistency. Types 6 and 7 are considered loose/liquid stools, or stools with a too low consistency.

The formation of short chain fatty acids and the subsequent lowering of pH inhibits the growth of pathogenic intestinal micro-organisms. The formation of short chain fatty acids, in particular butyric acid, results in the formation of mucus and/or food for enterocytes and hence in an increased gut barrier. The improvement of the microbiota may result in an improved immune system.

A further improvement besides functional effects on constipation and gut health can also lie in the field of product technological properties of young child formulas. For such formulas, fibres should be heat stable, should not have interaction with the minerals and decrease the mineral bioavailability. Also an increased water solubility will result in a better product stability and enable an increased concentration of fibres and improved effect of constipation with it.

Constipation (or obstipation), when referred to in the present invention is functional constipation. Rome IV diagnostic criteria for constipation are as follows for children of below 4 years of age:
The child should have at least 2 or more of the following symptoms for at least 1 months, with an absence of organic pathology to explain the symptoms:
- at most 2 bowel movements per week,
- at least 1 episode of incontinence per week, if toilet trained
- history of stool retention
- history of painful bowel movements
- large faecal mass present in the rectum
- history of clogging the toilet or large diameter stools
- symptoms such as early satiety, fussiness poor appetite that disappears immediately after having a bowel movement

Rome IV diagnostic criteria for constipation are as follows for children of 4 years of age or older:
The child should have at least 2 or more of the following symptoms for at least 1 months, with an absence of organic pathology to explain the symptoms:
- at most 2 bowel movements per week,
- at least 1 episode of incontinence per week
- history of volitional stool retention
- history of painful bowel movements
- large faecal mass present in the rectum
- history of clogging the toilet or large diameter stools
- does not meet criteria for irritable bowel syndrome

The present fibre mixture, or nutritional composition comprising the fibre mixture according to the invention, is intended for administration to healthy toddlers or healthy young children or adolescents from 1 year up to and including 12 years of age. The present fibre mixture, or nutritional composition comprising the fibre mixture, is for preventing constipation or for reducing the risk of constipation in such healthy subjects. In particular the present fibre mixture, or nutritional composition comprising the fibre mixture is beneficial for toddlers or young children from 1 year up to and including 12 years of age that are at risk of constipation, in particular functional constipation. In addition the present fibre mixture, or nutritional composition comprising the fibre mixture, is for treating constipation in toddlers or young children from 1 year up to and including 12 years of age that suffer from constipation. Preferably the subject suffering from constipation suffers from functional constipation. Preferably the subject suffering from constipation suffers from mild constipation. For all indications, preferably the present fibre mixture, or nutritional composition comprising the fibre mixture, is intended for administration to toddlers or young children from 1 year up to and including 12 years of age, more preferably from 1 year up to and including 6 years of age, even more preferably from 1 year up to and including 3 years of age.

In a preferred embodiment, for the purpose of prevention of constipation, the nutritional composition comprises between 0.2 and 2.5 g of the fibre mixture per 100 ml. In a preferred embodiment, for the purpose of treatment of constipation, the nutritional composition comprises between 1 and 10 g of the fibre mixture per 100 ml.

In one embodiment, the present fibre mixture, or nutritional composition comprising the fibre mixture, is for paediatric patients. In the context of the present invention, paediatric patients relate to toddlers and young children and adolescent human subjects from 1 up to and including 12 years of age who are under medical supervision for a disorder. More particular, the present fibre mixture, or nutritional composition comprising the fibre mixture, is for paediatric patients requiring nutritional support.

A preferred daily dosage of the fibre mixture according to the present invention is about 0.9-100 g per day, preferably about 1.2-50 g, more preferably 3-25 g, even more preferably 6-20 g fibre mixture per day.

When the nutritional composition according to the invention is in a liquid form, or reconstituted into its liquid form, the preferred volume of the nutritional composition that is administered on a daily basis is in the range of about 100 to 2500 ml, more preferably about 200 to 2000 ml per day, even more preferably about 300 to 500 ml per day.

### EXAMPLES

### Example 1: Fermentation of fibres by microbiota

For studying fermentation of fibres by microbiota the i-screen platform was used, see Schuren et al., The i-screen: A Versatile Preclinical Platform for Gut Microbiota Studies, J Prob Health 2019 7:212.

A standard human gut microbiota pool was used for the fermentation. This pool was established via fecal donations from 6 healthy volunteers (Caucasian individuals, age 25-60 years, no antibiotic use in the 3 months preceding the donation, self-assessment of health status). Before starting the i-screen fermentation with the test compounds, the standardized fecal pool was incubated in SIEM (standard ileal effluent medium) under anaerobic conditions overnight (37°C; 300 rpm) in order to activate the bacteria.

The fibres were tested at a concentration of 10 mg/ml. All compounds were tested in triplicate.

The following conditions were applied:
1 No added fibres, SIEM medium only
2 Mixture of beta-galactooligosaccharides (Vivinal GOS, Friesland Campina), inulin (Orafti HP (Beneo-Orafti Orey) Beneo BE), resistant starch (Novelose 330 (Ingredion UK Ltd) and non-soluble soy polysaccharides, source Fibrim 2000 (DuPont/Danisco) in a weight ratio of 38:38:4:20, prior art MF4
3 Mixture of beta-galactooligosaccharides (Vivinal GOS, Friesland Campina), inulin (Orafti HP (Beneo-Orafti Orey) Beneo BE), resistant starch (Novelose 330 (Ingredion UK Ltd) and soluble soy polysaccharides, source Soyafibe-S-Ca100 (Fuji Oil Co, Ltd), in a weight ratio of 38:38:4:20, new MF4
4 Lactulose (Duphalac Lactulose, Solvay, the Netherlands)

After 24 hours of fermentation in SIEM medium at 37°C, samples were collected for pH measurements, DNA isolation and for metabolite analysis. The remaining material was stored at -20°C.

DNA was isolated as described by Ladirat et al. J Microbiol Meth 2013, 92(3), 387-397 with some minor adjustments: the samples were initially mixed with 300 µl lysis buffer (Agowa, Berlin, Germany), 500 µl zirconium beads (0.1 mm), and 500 µl phenol, before being introduced to a Bead Beater (BioSpec Products, Bartlesville, OK, USA) for 3 min. 16S amplicon sequencing DNA was analyzed by using 16S rDNA amplicon sequencing. The V4 hypervariable region was targeted. 100 pg of DNA was amplified as described by Kozich et al. AEM 2013, 79(17): 5112-5120, with the exception that 30 cycles were used instead of 35, applying F515/R806 primers (Caporaso et al., Proc Nat Acad Sci 2011, 108: 4516-4522). Primers included Illumina adapters and a unique 8- nt sample index sequence key. The amplicon libraries were pooled in equimolar amounts and purified using the QIAquick Gel Extraction Kit (QIAGEN). Amplicon quality and size were analyzed on a Fragment Analyzer (Advanced Analytical Technologies, Inc.). Paired-end sequencing of amplicons (approximately 400 base pairs) was conducted on the Illumina MiSeq platform (Illumina, Eindhoven, The Netherlands). SCFA covering acetate, propionate, and n-butyrate and branched chain fatty acids (BCFA) covering iso-butyrate and iso-valerate were analyzed as described by as described by Van Nuenen Microbiology in Health and Disease 2003, 15: 137-144. Briefly, fermented material from the i-screen samples was centrifuged (-12,000 g, 5 min). Cells were removed from the supernatant by filter sterilization (0.45 µm). A mixture of formic acid (20%), methanol and 2-ethyl butyric acid (internal standard, 2 mg/ml in methanol) was added. A 3 µl sample with a split ratio of 75.0 was injected on a GC-column (ZB5HT inferno, ID 0.52 mm, film thickness 0.10 µm; Zebron; Phenomenex, USA) in a Shimadzu GC-2014 gas chromatograph. For the analysis of total lactate, material resulting from the i-screen fermentation was sampled and centrifuged at 4,000 g for 5 minutes. The supernatant was filter sterilized using 0.45 µm filters. The D- and L-lactate were determined by the Arena 20XL analyser. The principle of the assay is the enzyme-catalyzed reaction of lactic acid + NAD→ pyruvate + NADH. The amount of NADH generated is stoichiometrically linked to the amount of lactic acid present and is measured by the amount of light absorbed by NADH at wavelength 340 nm. By using both the L-lactate dehydrogenase and D-lactate dehydrogenase enzymes in one reaction, the concentration of the total amount of lactic acid (i.e. both L-lactic acid and D-lactic acid) is determined. The assay is based on the D- and L-lactic acid assay kits (Thermo Fisher Scientific OY, www.e-labeling.eu/TSF984306IFU and www.e-labeling.eu/TSF984308IFU). The assay was performed following the instructions of the manufacturer with one adaptation: the reagents DLac R1 and LLac R1 from the two kits were mixed in a 1:1 ratio to detect both L and D isomers of lactic acid. Calibration of the assay was done with an 'Acid combination standard' containing both L-lactic acid and D-lactic acid.

Both MF4 fibres mixes lowered the pH and showed the formation of SCFA when compared with the blanc. Isobutyrate and isovalerate was reduced to below detection limits in both MF4 mixes when compared with the blanc. Lactic acid levels at 24 h were below detection levels. The new fibre mix was improved in that is showed a lower pH, a higher SCFA production when compared with the prior art MF4 mix. The amount of acetate was comparable. Unexpectedly the amount of butyrate formed was statistically significantly higher in the new MF4 mix, and the amount of propionic acid was significantly lower, see table 1. This is indicative for an improved effect on constipation.

**Table 1: pH and SCFA formation after 24 h of fermentation of fiber mixes**

| | Mean pH mean (s.d.) | Mean Total SCFA mM | Mean Acetate mM (s.d.) | Mean Propionate mM (s.d.) | Mean Butyrate mM (s.d.) |
|---|---|---|---|---|---|
| MF4prior art | 5.47 (0.06) | 92.57 (0.72) | 39.01 (0.60) | 4.99 (0.14) | 48.57 (0.26) |
| MF4new | 5.50 (0.0) | 98.84 (2.16) | 40.74 (2.26) | 4.58 (0.09)* | 53.51 (1.04)* |
| lactulose | 5.57 (0.03) | 100.9 (2.67) | 44.93 (1.40) | 6.61 (0.12) | 49.39 (1.16) |

| | | | | | |
|---|---|---|---|---|---|
| *p < 0.05 when compared with prior art MF4 or lactulose, Anova, Tukey HSD | | | | | |

### Effect microbiota

The microbiota was studied on a phylum level. It was found that levels of Actinobacteria, to which the bifidobacteria belong, were not statistically different between the two MF4 mixes.

On genus level the number of Bifidobacteria was not significantly different between prior art MF4 and new MF4. In general the microbiota composition did not differ significantly between the two mixes and was rich in beneficial bacteria and low in potentially pathogenic bacteria.

The new MF4 mixture with the soluble soy fibre thus stimulated the formation of a beneficial microbiota.

So when the effect of the fibre mixtures on SCFA, butyrate levels and the microbiota composition is taken into account the mixture with beta-galactooligosaccharides, inulin, resistant starch and soluble soy polysaccharides is superior when compared to a mixture with insoluble soy polysaccharides or when compared to a mixture without soy polysaccharides.

### Example 2: Young child milk

A packed powder with on the pack containing instructions to reconstitute the powder with water to form a ready to drink formula with the following composition per 100 ml:
- 67 kcal
- 2.6 g fat (mix of vegetable oils and fish oil)
- 1.3 g protein (casein and whey protein from cow's milk)
- 8.7 g digestible carbohydrates (mainly lactose)
- 2.0 g fibre per 100 ml: with beta-galactooligosaccharides / inulin / resistant starch / soluble soy polysaccharides in a 38/38/4/20 wt/wt ratio
- minerals, trace elements, vitamins and other micro-nutrients as known and in line with regulations.

The formulation of such young child formula is known to the skilled person. The product is labelled to be for young children with an age of 1 to 3 years As a source of beta-galactooligosaccharides Vivinal^{®} GOS is used (FrieslandCampina, Domo-Borculo NL), as a source of inulin Orafti^{®} HP (Beneo-Orafti Orey) Beneo BE) is used, as a source of resistance starch Novelose^{®}330 (Ingredion UK Ltd.) is used, and as a source of soluble soy polysaccharide Soyafibe-S-Ca100^{®} (Fuji Oil Co, Ltd) is used.

## Claims

1. A fibre mixture consisting of beta-galactooligosaccharides, inulin, resistant starch, and soluble soy polysaccharides, wherein the weight ratio beta-galactooligosaccharides : inulin : resistant starch : soluble soy polysaccharides is about 38 : 38 : 4 : 20.

2. The fibre mixture according to claim 1, wherein the inulin has an average degree of polymerization above 15, preferably above 20.

3. A nutritional composition, when in liquid form comprising 0.4 - 10 g of the fibre mixture according to any one of claims 1-2 per 100 ml, or when in powder form, comprising 3 - 45 g of the fibre mixture according to any one of claims 1-2 per 100 g dry weight, wherein the fibres present in the nutritional composition consist of the fibre mixture according to any one of claims 1-2.

4. The nutritional composition according to claim 3 comprising lipid, protein and digestible carbohydrates, preferably when in liquid form comprising 1.5 - 4.5 g lipid per 100 ml, 0.8 - 2.5 g protein per 100 ml and 5 - 15 g digestible carbohydrates per 100 ml, and preferably when in powder form comprising 10 - 35 g lipid per 100 g, 6 - 20 g protein per 100 g and 35 - 84 g digestible carbohydrates per 100 g dry weight.

5. The nutritional composition according to any one of claims 3-4, which is a young child formula.

6. The nutritional composition according to any one of claims 3-5, which is a powder suitable to be reconstituted with water to a liquid nutritional composition.

7. The nutritional composition according to any one of claims 3-6, for use in treating or preventing constipation in a human subject.

8. Non-therapeutic use of the nutritional composition according to any one of claims 3-6, for softening stool and/or decreasing stool consistency and/or maintaining a soft stool in a human subject, compared to stool consistency resulting from the fibre mixture wherein the soluble soy polysaccharides are replaced by insoluble soy polysaccharides, wherein stool consistency is quantified by using the Bristol Stool Form Scale (Lewis & Heaton, Scand J Gastroenterol 1997, 32:920-924).

9. Non-therapeutic use of the nutritional composition according to any one of the claims 3-6, for increasing intestinal butyrate levels and/or for decreasing intestinal propionate levels, preferably for increasing intestinal butyrate levels, compared to the levels of butyrate and/or propionate resulting from the fibre mixture wherein the soluble soy polysaccharides are replaced by insoluble soy polysaccharides.

10. The nutritional composition for use or non-therapeutic use according to any one of claims 7-9, wherein the human subject has an age of 1 to 12 years, preferably 1 to 6 years, more preferably 1 to 3 years.

## Patentansprüche

1. Ballaststoffmischung, die aus Beta-Galactooligosacchariden, Inulin, resistenter Stärke und löslichen Sojapolysacchariden besteht, wobei das Gewichtsverhältnis Beta-Galactooligosaccharide : Inulin : resistente Stärke : lösliche Sojapolysaccharide etwa 38 : 38 : 4 : 20 beträgt.

2. Ballaststoffmischung nach Anspruch 1, wobei das Inulin einen durchschnittlichen Polymerisationsgrad über 15, vorzugsweise über 20 aufweist.

3. Ernährungszusammensetzung, die in flüssiger Form 0,4 - 10 g der Ballaststoffmischung nach einem der Ansprüche 1-2 pro 100 ml umfasst, oder in Pulverform 3 - 45 g der Ballaststoffmischung nach einem der Ansprüche 1-2 pro 100 g Trockengewicht umfasst, wobei die in der Ernährungszusammensetzung vorhandenen Ballaststoffe aus der Ballaststoffmischung nach einem der Ansprüche 1-2 bestehen.

4. Ernährungszusammensetzung nach Anspruch 3, die Lipid, Protein und verdauliche Kohlenhydrate umfasst, die vorzugsweise in flüssiger Form 1,5 - 4,5 g Lipid pro 100 ml, 0,8 - 2,5 g Protein pro 100 ml und 5 - 15 g verdauliche Kohlenhydrate pro 100 ml umfasst und vorzugsweise in Pulverform 10 - 35 g Lipid pro 100 g, 6 - 20 g Protein pro 100 g und 35 - 84 g verdauliche Kohlenhydrate pro 100 g Trockengewicht umfasst.

5. Ernährungszusammensetzung nach einem der Ansprüche 3-4, die eine Kleinkindnahrung ist.

6. Ernährungszusammensetzung nach einem der Ansprüche 3-5, die ein Pulver ist, das mit Wasser zu einer flüssigen Ernährungszusammensetzung rekonstituiert werden kann.

7. Ernährungszusammensetzung nach einem der Ansprüche 3-6 zur Verwendung bei der Behandlung oder Vorbeugung von Verstopfung bei einem menschlichen Patienten.

8. Nicht-therapeutische Verwendung der Ernährungszusammensetzung nach einem der Ansprüche 3-6 zur Erweichung des Stuhls und/oder Verringerung der Stuhlkonsistenz und/oder Aufrechterhaltung eines weichen Stuhls bei einem menschlichen Patienten im Vergleich zu der Stuhlkonsistenz, die sich aus der Ballaststoffmischung ergibt, in der die löslichen Sojapolysaccharide durch unlösliche Sojapolysaccharide ersetzt sind, wobei die Stuhlkonsistenz unter Verwendung der Bristol Stool Form Scale (Lewis & Heaton, Scand J Gastroenterol 1997, 32:920-924) quantifiziert wird.

9. Nicht-therapeutische Verwendung der Ernährungszusammensetzung nach einem der Ansprüche 3-6 zur Erhöhung intestinaler Butyratspiegel und/oder zur Senkung intestinaler Propionatspiegel, vorzugsweise zur Erhöhung intestinaler Butyratspiegel im Vergleich zu Butyratspiegeln und/oder Propionatspiegeln, die sich aus der Ballaststoffmischung ergeben, in der die löslichen Sojapolysaccharide durch unlösliche Sojapolysaccharide ersetzt sind.

10. Ernährungszusammensetzung zur Verwendung oder nicht-therapeutischen Verwendung nach einem der Ansprüche 7-9, wobei der menschliche Patient ein Alter von 1 bis 12 Jahren, vorzugsweise von 1 bis 6 Jahren, besonders bevorzugt von 1 bis 3 Jahren hat.

## Revendications

1. Mélange de fibres composé de bêta-galacto-oligosaccharides, d'inuline, d'amidon résistant et de polysaccharides de soja solubles, où le rapport pondéral bêta-galacto-oligosaccharides : inuline : amidon résistant : polysaccharides de soja solubles est d'environ 38 : 38 : 4 : 20.

2. Mélange de fibres selon la revendication 1, où l'inuline a un degré moyen de polymérisation supérieur à 15, de préférence supérieur à 20.

3. Composition nutritionnelle, quand sous forme liquide comprenant de 0,4 à 10 g du mélange de fibres selon l'une quelconque des revendications 1 à 2 pour 100 ml, ou quand sous forme de poudre, comprenant de 3 à 45 g du mélange de fibres selon l'une quelconque des revendications 1 à 2 pour 100 g de poids sec, où les fibres présentes dans la composition nutritionnelle sont constituées du mélange de fibres selon l'une quelconque des revendications 1 à 2.

4. Composition nutritionnelle selon la revendication 3 comprenant des lipides, des protéines et des glucides digestibles, de préférence quand sous forme liquide comprenant de 1,5 à 4,5 g de lipides pour 100 ml, de 0,8 à 2,5 g de protéines pour 100 ml et de 5 à 15 g de glucides digestibles pour 100 ml, et de préférence quand sous forme de poudre comprenant de 10 à 35 g de lipides pour 100 g, de 6 à 20 g de protéines pour 100 g et de 35 à 84 g de glucides digestibles pour 100 g de poids sec.

5. Composition nutritionnelle selon l'une quelconque des revendications 3 à 4, qui est une formule pour enfant en bas âge.

6. Composition nutritionnelle selon l'une quelconque des revendications 3 à 5, qui est une poudre pouvant être reconstituée avec de l'eau en une composition nutritionnelle liquide.

7. Composition nutritionnelle selon l'une quelconque des revendications 3 à 6, pour l'utilisation dans le traitement ou la prévention de la constipation chez un sujet humain.

8. Utilisation non thérapeutique de la composition nutritionnelle selon l'une quelconque des revendications 3 à 6, pour ramollir les selles et/ou diminuer la consistance des selles et/ou maintenir des selles molles chez un sujet humain, par rapport à la consistance des selles résultant du mélange de fibres où les polysaccharides de soja solubles sont remplacés par des polysaccharides de soja insolubles, où la consistance des selles est quantifiée en utilisant l'échelle de forme des selles de Bristol (Lewis & Heaton, Scand J Gastroenterol 1997, 32:920-924).

9. Utilisation non thérapeutique de la composition nutritionnelle selon l'une quelconque des revendications 3 à 6, pour augmenter les niveaux de butyrate intestinal et/ou pour diminuer les niveaux de propionate intestinal, de préférence pour augmenter les niveaux de butyrate intestinal, par rapport aux niveaux de butyrate et/ou de propionate résultant du mélange de fibres où les polysaccharides de soja solubles sont remplacés par des polysaccharides de soja insolubles.

10. Composition nutritionnelle pour l'utilisation ou l'utilisation non thérapeutique selon l'une quelconque des revendications 7 à 9, où le sujet humain a un âge de 1 à 12 ans, de préférence de 1 à 6 ans, plus préférablement de 1 à 3 ans.
